(19) **Europäisches Patentamt European Patent Office Office européen des brevets**

(11) **EP 3 761 955 B1**

(12) **EUROPEAN PATENT SPECIFICATION**

(45) Date of publication and mention of the grant of the patent:
**08.10.2025  Bulletin 2025/41**

(21) Application number: **19724635.8**

(22) Date of filing: **05.03.2019**

(51) International Patent Classification (IPC):
**A61K 9/00** (2006.01)   **A61K 9/16** (2006.01)
**A61K 47/69** (2017.01)

(52) Cooperative Patent Classification (CPC):
**A61K 47/6939; A61K 9/0019; A61K 9/16**

(86) International application number:
**PCT/PL2019/050014**

(87) International publication number:
**WO 2019/172790 (12.09.2019 Gazette 2019/37)**

(54) **ANTHRACYCLINE ENCAPSULATED WITH POLYSACCHARIDE FOR USE IN THE TREATMENT OF TUMOURS**

ANTHRACYCLIN EINGEKAPSELT MIT POLYSACCHARID ZUR VERWENDUNG BEI DER BEHANDLUNG VON TUMOREN

ANTHRACYCLINE ENCAPSULÉE AVEC UN POLYSACCHARIDE DESTINÉE À ÊTRE UTILISÉE DANS LE TRAITEMENT DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.03.2018  PL 42477318**

(43) Date of publication of application:
**13.01.2021  Bulletin 2021/02**

(73) Proprietor: **Nanovelos S.A.**
**02-532 Warszawa (PL)**

(72) Inventors:
- **WASIAK, Iga**
  **01-912 Warszawa (PL)**
- **KICIAK, Adam**
  **00-791 Warszawa (PL)**
- **CIACH, Tomasz**
  **02-765 Warszawa (PL)**
- **KULIKOWSKA-DARLAK, Aleksandra**
  **08-430 Huta  Zelechowska (PL)**
- **PIETRZAK, Piotr**
  **01-250 Warszawa (PL)**
- **SOBIECKA, Agnieszka**
  **01-114 Warszawa (PL)**
- **KOSNIK, Wioletta**
  **07-311 Wasewo (PL)**

- **PIETRAS, Joanna**
  **02-518 Warszawa (PL)**
- **ZERO, Pawel**
  **01-184 Warszawa (PL)**
- **ZUK, Pawel**
  **01-318 Warszawa (PL)**
- **MALKOWSKA, Justyna**
  **07-100 Wegrów (PL)**
- **ADAMSKA, Kinga**
  **96-321 Nowa Bukówka (PL)**
- **CHROMINSKI, Mikolaj**
  **07-320 Malkinia Górna (PL)**

(74) Representative: **Grzelak, Anna**
**Sigeon IP, sp z o. o.**
**ul. Bukowinska 24A m. 65**
**02-703 Warszawa (PL)**

(56) References cited:
**KR-A- 20170 093 400     US-A1- 2011 104 052**
**US-A1- 2016 279 064**

- **IGA WASIAK ET AL: "Dextran Nanoparticle Synthesis and Properties", PLOS ONE, vol. 11, no. 1, 11 January 2016 (2016-01-11), pages e0146237, XP055602444, DOI: 10.1371/ journal.pone.0146237**

EP 3 761 955 B1

**(Cont. next page)**

- **AGARWAL A ET AL: "Dextran conjugated dendritic nanoconstructs as potential vectors for anti-cancer agent", BIOMATERIALS, ELSEVIER SCIENCE PUBLISHERS BV., BARKING, GB, vol. 30, no. 21, 1 July 2009 (2009-07-01), pages 3588 - 3596, XP026128025, ISSN: 0142-9612, [retrieved on 20090402], DOI: 10.1016/ J.BIOMATERIALS.2009.03.016**

**Description**

## TECHNICAL FIELD

The invention relates to a new form of a drug in the form of anthracycline encapsulated with a polysaccharide, selected from epirubicin, daunorubicin, doxorubicin, idarubicin, especially encapsulated with dextran, for use in the treatment of specific tumours. The new form of drug administration allows to increase the relative amount of chemotherapeutic agent administered, improve its targeting to the site of the desired effect while ensuring lower toxicity for the body, which makes it possible to increase the effectiveness of anti-tumour (anti-cancer) therapy carried out using an anthracycline encapsulated with a polysaccharide.

## STATE OF THE ART

Anthracyclines belong to the group of anticancer antibiotics and are widely used as low-dose chemotherapeutic drugs for various tumours, in particular, leukaemia, metastatic breast cancer, ovarian cancer and colon and rectal cancer. Anthracyclines interfere with the functioning of essential cellular processes, including DNA reading and replication and cell protein synthesis. These drugs include doxorubicin, daunorubicin, idarubicin, epirubicin, dactinomycin and bleomycin. Anthracyclines have a very similar chemical structure. Anthracyclines are among the most active groups of chemotherapeutic drugs. They are highly effective against a spectrum of tumours, including both haematological malignancies and solid tumours (lymphoma, gastric cancer, non-small cell lung cancer, sarcoma, breast cancer). Among the many derivatives produced, several anthracyclines are currently used as drugs, including doxorubicin (DOX), daunorubicin (DAU), epirubicin (EPI) and idarubicin (IDA) [see literature, item 200].

Clinical use of anthracyclines is limited by the development of tumour cell resistance and toxicity to healthy tissues. Particularly severe side effects of anthracycline administration include nausea and vomiting, mucositis, stomatitis, hair loss, myelosuppression, which are for the most part reversible. Bone marrow suppression, although reversible, predisposes the patient to severe complications, such as infections during treatment. Administration of anthracyclines also causes irreversible side effects due to their high toxicity: soft tissue necrosis at the administration site in case of unintentional extravascular administration as well as high cardiotoxicity (in particular in the form of chronic congestive cardiomyopathy and heart failure). High cardiotoxicity poses a particularly high risk when administering anthracyclines in their current form, as free radicals produced by anthracyclines cause peroxidation of the sarcoplasmic reticulum of the heart, leading to the $Ca^{2+}$-mediated necrosis of the myocardium. Said toxicity is selective for cardiac tissue because the catalase able to neutralise free radicals is not present in cardiac tissue [see literature, item 207].

To limit side effects, it was established that the maximum recommended cumulative doses of anthracyclines DAU and DOX should amount to 500 or 450 to 600mg/m$^2$, respectively [see literature, item 201].

In various situations individual anthracyclines can be used interchangeably; however, as a rule [see literature, item 202]:

- doxorubicin (DOX) is used to treat breast tumours, breast cancer, paediatric solid tumours, Wilms tumour, sarcoma, soft tissue sarcoma, Ewing sarcoma, non-Hodgkin's lymphoma, aggressive lymphoma, lymphatic leukaemia, myeloblastic leukaemia, acute leukaemia, multiple myeloma, Hodgkin's disease, endometrial cancer, small cell lung cancer, gastric cancer, papillary and follicular thyroid cancer, bladder cancer, osteosarcoma, neuroblastoma,
- daunorubicin (DAU) is used to treat leukaemia, particularly acute lymphoblastic leukaemia and myeloid leukaemia,
- idarubicin (IDA) is used to treat leukaemia, especially acute myeloid leukaemia and acute lymphatic leukaemia and breast cancer;
- epirubicin (EPI) is used to treat breast cancer, lymphoma, including malignant lymphoma, non-Hodgkin's lymphoma, sarcoma, including soft tissues, ovarian cancer, leukaemia, small cell lung cancer, gastric tumour, bladder tumour.

Patent PL221351 discloses a method for obtaining nanoparticles from polysaccharides and their derivatives, which are carriers of active substances due to their specific partial oxidation to form aldehyde groups and the attachment of compounds containing an amine or other group with an R-NH2 binding reacting with aldehyde groups, wherein the active substance containing an amine, amide or hydrazide group may be a drug, for example, daunorubicin, doxorubicin. Accordingly, PL221351 discloses a method for producing anthracyclines encapsulated with a polysaccharide.

Patent KR 2017 0093400 A discloses dextran beads comprising an anthracycline anticancer agent such as Doxorubicin hydrochloride, Epirubicin hydrochloride, Idarubicin, Daunorubicin.

Iga Wasiak ET AL ("Dextran Nanoparticle Synthesis and Properties",PLOS ONE, vol. 11) discloses dextran nanoparticles encapsulating doxorubicin, the nanoparticles are used for drug carrier, the diameter of the nanoparticles is between 90 and 120 nm.

AGARWAL A ET AL ("Dextran conjugated dendritic nanoconstructs as potential vectors for anti-cancer agent",BIOMATERIALS, vol. 30, pages 3588-3596) discloses dendrimers comprising dextran and doxorubicin. The dendrimers allow good performance and safety of the drug but reduced side effects and toxicity.

The first drug authorised for medical use (FDA 1995) based on nanoparticles was a combination of liposomes and doxorubicin. Since then, several combinations of nanoparticles with anthracycline drugs have been authorised for therapeutic use. However, all solutions are based on liposomes, and they only involve two drugs from this group: doxorubicin and daunorubicin [see literature, item 208].

The efficacy of anthracyclines in the treatment of tumours, especially doxorubicin, daunorubicin, idarubicin, epirubicin, is reduced due to the lack of targeted drug delivery to tissues with neoplastic lesions and low therapeutic index as well as relatively high toxicity.

## SUMMARY OF THE INVENTION

The invention relates to an anthracycline encapsulated with a polysaccharide for use in the treatment of tumour, characterised in that the anthracycline is epirubicin the tumour is ovarian cancer and polysaccharide is dextran.

In a preferable embodiment, the anthracycline encapsulated with a polysaccharide for tumour treatment, anthracycline particles used for tumour treatment have an average size in the range of 10-500 nm, more preferably 50-200 nm, most preferably 70-160 nm (diameters in a hydrated state).

In said embodiment of the invention, anthracyclines encapsulated with a polysaccharide can be used for monotherapy, combined therapy, simultaneous or subsequent use in anti-tumour therapy. A known chemotherapeutic agent, e.g. cis-platinum or a radiotherapeutic agent may be an additional auxiliary component. Therapy of this kind reduces undesired side effects.

In general, anthracyclines encapsulated with a polysaccharide are intended for intravenous administration, although in specific embodiments, e.g. for the treatment of bladder cancer, they may be used as intravesical infusions.

Anthracyclines encapsulated with a polysaccharide in use for tumour treatment according to the invention in the form of finished injectable formulations comprise aqueous and non-aqueous isotonic sterile solutions, optionally containing antioxidants, buffers, isotonic additives and the like. A typical carrier is, e.g. injection water or saline solution.

In general, the formulation for final use containing anthracyclines encapsulated with a polysaccharide for use in tumour treatment according to the invention is obtained by diluting or mixing them with a carrier or diluent.

The dose of anthracyclines encapsulated with a polysaccharide for use in tumour treatment of the invention is determined taking into account the type of tumour, type of therapy, patient's age, patient's weight, approximate body surface of the patient, as well as other special circumstances such as liver parameters, kidneys, cardiac history etc. A person skilled in the art will be able to determine the right dose for a particular patient. However, the single dose administered should not exceed the maximum tolerated dose established for a specific anthracycline encapsulated in a specific polysaccharide.

## BRIEF DESCRIPTION OF THE FIGURES

To be better understood, the invention, the scope of the invention is limited to epirubicin encapsulated with dextran, been illustrated in embodiments and the accompanying figures, where:

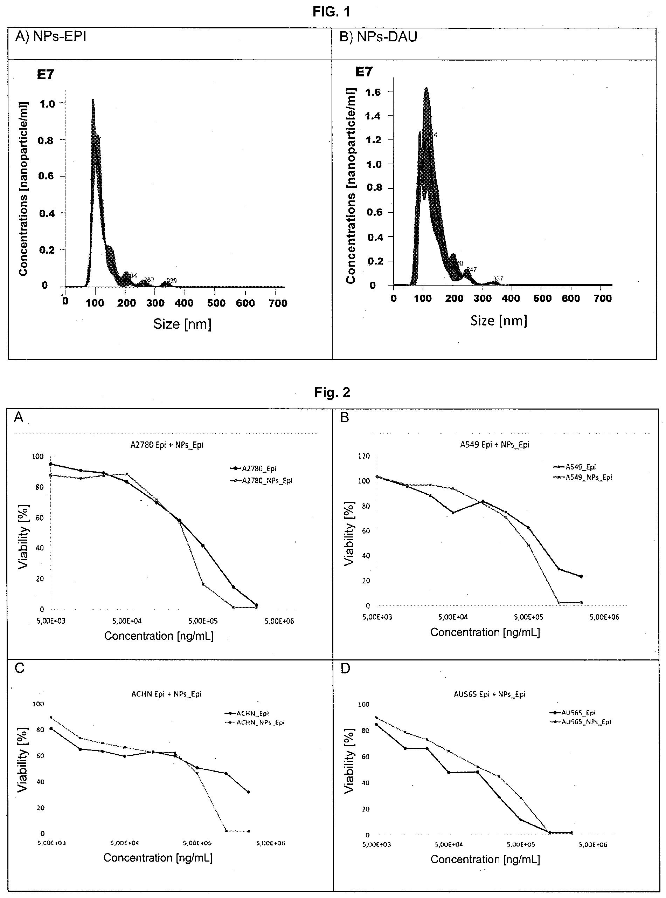

**Fig.1** shows the distribution of diameters of the obtained nanoparticles with the drug **(A)** NPs_EPI, **B)** NPs-DAU. Diameter distribution obtained after repeated rehydration of nanoparticles in injection water after 3h.

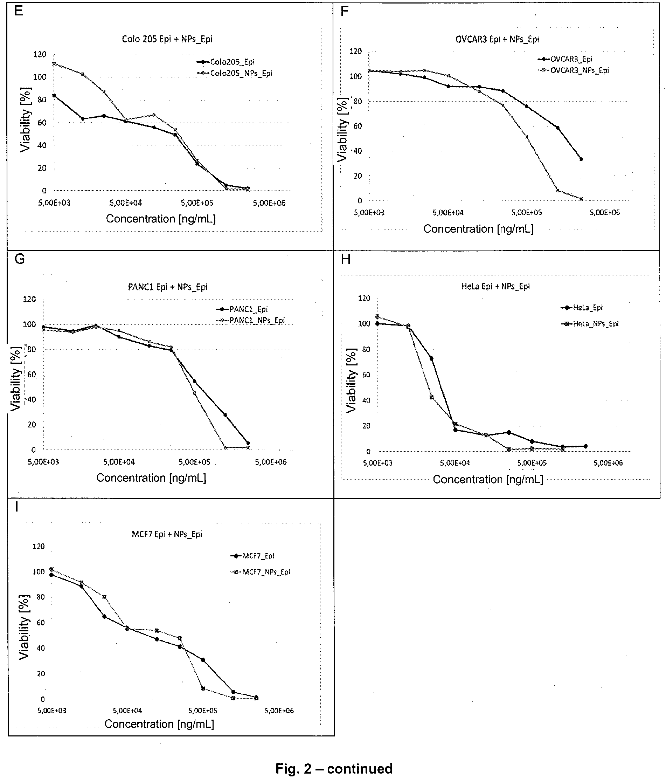

**Fig. 2** is a comparison of cytotoxicity to selected tumour lines. Nanoparticles with encapsulated epirubicin on lines **A)** A2780, **B)** A549, **C)** ACHN, **D)** AU565, **E)** COLO205, **F)** OVCAR-3, **G)** PANC1, **H)** HeLa and **I)** MCF7. The designation Epi- refers to epirubicin administered alone, NPs_Epi is the administration of dextran-encapsulated epirubicin.

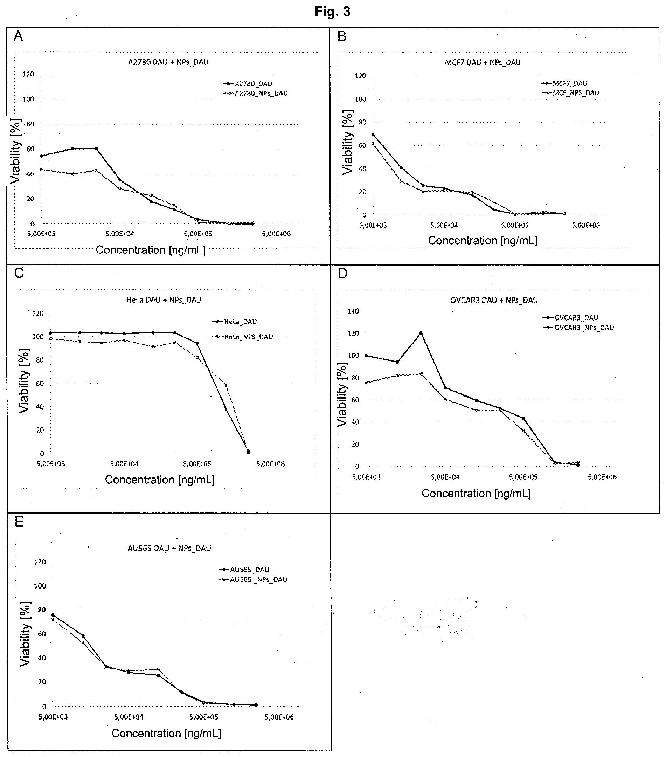

**Fig. 3** is a comparison of cytotoxicity to selected tumour lines. Nanoparticles with encapsulated daunorubicin on lines **A)** A2780, **B)** MCF7 **C)** HeLa, **D)** OVCAR-3, **E)** AU565. The designation DAU- refers to daunorubicin administered alone, NPs_DAU is the administration of dextran-encapsulated daunorubicin.

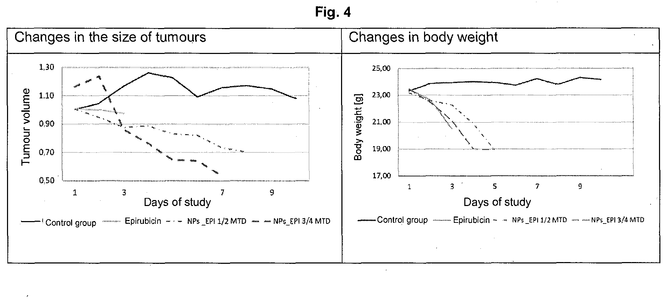

**Fig.4** The graph shows changes in tumour size and body weight in mice during the anti-tumour efficacy determination of encapsulated epirubicin (NPs_Epi) in dextran nanoparticles, administered in two doses: 1/2 MTD (dose of 3.75 mg/kg bw) and 3/4 MTD (22.5 mg/kg bw) compared to the control (receiving water for injections) and administration of non-encapsulated Epirubicin (dose of 15 mg/kg bw).

## EMBODIMENTS OF THE INVENTION

The following examples are provided solely to illustrate the invention and for clarifying the individual aspects thereof, and not to limit it, and should not be considered to be equivalent to the total scope thereof, which is defined in the appended claims. In the examples below, unless otherwise indicated, standard materials and methods were employed as used in the art, or it was proceeded according to the manufacturer's recommendations for particular materials and methods.

## EXAMPLES

## Example 1

**Production of anthracyclines encapsulated with a polysaccharide**

### *(a) Preparation of dextran-encapsulated epirubicin (NPs_EPI)*

Dextran-encapsulated epirubicin was prepared according to the preparation method of nanoparticles from polysaccharides as described in patent PL221251 (see in particular Examples 2 and 4) using dextran with a molecular weight of 70 kDa (oxidation degree 5-15%) and dodecylamine hydrochloride. The substitution degree of aldehyde groups produced in dextran by winding agent dodecylamine is 10 - 20%. The substitution degree of aldehyde groups produced in dextran by epirubicin is 4 - 10%. The other generated aldehyde groups were substituted with alanine. Nanoparticles were prepared with an average size between 80 and 140 nm (**Fig. 1 A**) as measured in aqueous solutions using NanoSight LM 10 (405 nm laser). The determined epirubicin content in a dry matter of nanoparticles is 3.0 - 5%. The obtained nanoparticles were freeze-dried and stored in sealed containers at the temperature of 4°C. Before the tests, the nanoparticles were again rehydrated (suspended) for 3h in injection water.

### *(b) Preparation of dextran-encapsulated daunorubicin (NPs_DAU)* (comparative nanoparticles, not part of the invention)

Dextran-encapsulated daunorubicin was prepared according to the preparation method of nanoparticles from polysaccharides as described in patent PL221251 (see in particular Examples 2 and 4) using Dextran with a molecular weight of 70 kDa (oxidation degree 5-15%) and dodecylamine hydrochloride. The substitution degree of aldehyde groups produced in dextran by winding agent dodecylamine is 10 - 20%. The substitution degree of aldehyde groups produced in dextran by daunorubicin is 4 - 10%. The other generated aldehyde groups were substituted with alanine. Nanoparticles were produced with an average size between 80 and 140 nm (**Fig. 1B**) as measured in aqueous solutions using NanoSight LM 10 (405 nm laser). The determined daunorubicin content in a dry matter of nanoparticles is 3.0 - 5%. The obtained nanoparticles were freeze-dried and stored in sealed containers at the temperature of 4°C. Before the tests, the nanoparticles were again rehydrated (suspended) for 3h in injection water.

### *(c) Preparation of dextran-encapsulated doxorubicin (NPs_DOX)* (comparative nanoparticles, not part of the invention)

Dextran-encapsulated doxorubicin was prepared according to the preparation method of nanoparticles from polysaccharides as described in patent PL221251 (see in particular Examples 2 and 4) using Dextran with a molecular weight of 70 kDa (oxidation degree 5-15%) and dodecylamine hydrochloride. The substitution degree of aldehyde groups produced in dextran by winding agent dodecylamine is 10 - 20%. The substitution degree of aldehyde groups produced in dextran by doxorubicin is 4 - 10%. The other generated aldehyde groups were substituted with alanine. Nanoparticles of 80-150 nm were produced as measured in aqueous solutions using NanoSight LM 10 (405 nm laser). The determined doxorubicin content in a dry matter of nanoparticles is 3.0 - 5 %. The obtained nanoparticles were freeze-dried and stored in sealed containers at the temperature of 4°C. Before the tests, the nanoparticles were again rehydrated (suspended) for 3h in injection water.

### *(d) Preparation of dextran-encapsulated idarubicin (NPs_IDA)* (comparative nanoparticles, not part of the invention)

Dextran-encapsulated idarubicin was prepared according to the preparation method of nanoparticles from polysaccharides as described in patent PL221251 (see in particular Examples 2 and 4) using Dextran with a molecular weight of 70 kDa (oxidation degree 5-15%) and dodecylamine hydrochloride. The substitution degree of aldehyde groups produced in dextran by winding agent dodecylamine is 10 - 20%. The substitution degree of aldehyde groups produced in dextran by idarubicin is 4 -10%. The other generated aldehyde groups were substituted with alanine. Nanoparticles were produced with an average size of 80 - 150 nm, as measured in aqueous solutions using NanoSight LM 10 (405 nm laser). The determined idarubicin content in a dry matter of nanoparticles is 3.0 - 5 %. The obtained nanoparticles were freeze-dried and stored in sealed containers at the temperature of 4°C. Before the tests, the nanoparticles were again rehydrated (suspended) for 3h in injection water.

A similar method was used to prepare nanoparticles encapsulated with another polysaccharide: cellulose, amylose, starch and heparin, encapsulating: epirubicin, daunorubicin, doxorubicin and idarubicin. A similar method was used to obtain nanoparticles from particular polysaccharides dextran and cellulose, amylose, starch, heparin, which contained no anthracyclines, and which served as controls in further studies.

### Example 2

**Determination of cytotoxicity of anthracyclines encapsulated with a polysaccharide on cell lines (nanoparticles combined with the drug)**

The invention is limited to epirubicin encapsulated into dextran nanoparticles.

The subject of the study was to determine the cytotoxicity of a combination of anthracyclines encapsulated with a polysaccharide EPI, DAU, DOX, IDA on cell lines. Toxicity was assessed using a quantitative method based on the colourimetric technique (MTT) [see literature item 203]. In this test, amber dehydrogenase present in the cells converts the soluble tetrazolium salt (3-4,5-dimethylthiazol-2-yl)-2,5-diphenyltetrazolium bromide) into a reduced form. The reaction yields water-insoluble purple crystalline formazan. The number of crystals formed depends on the enzyme activity so that it is directly proportional to the number of viable cells in the sample. Spectrophotometric measurement requires the use of an organic solvent to dissolve the crystals obtained (isopropanol). The change in colour intensity is measured by spectrophotometry at 570 nm wavelength. The test was performed for a number of dilutions (9) of nanoparticle solutions containing the tested anthracycline. The baseline concentration of nanoparticles for MTT tests was 2.5 mg of nanoparticles/ml. The results were compared to cytotoxicity results obtained for pure drugs with equimolar drug concentration (equal to the concentration encapsulated in nanoparticles). The study was also performed for drug-free nanoparticles (the carrier itself was obtained similarly to the nanoparticles with the drug). Cells not exposed to toxic substances were used as a control for toxicity studies.

*Study protocol* [see literature items 204, 205].

After reaching 85-90% confluence, the cells were trypsinised using a 0.25% trypsin solution. The cells were then centrifuged (1200 rpm, 5 min.) and suspended in a culture medium suitable for a particular cell line. The medium did not contain phenolic red. The cells were plated at a concentration of 1000 cells/100 $\mu$l of medium per culture well (96-well plates). The prepared plates were incubated (37°C/5%$CO_2$/22h to 26 h). Subsequently, the culture medium was replaced with a fresh one (100 $\mu$l) containing the appropriate concentration of the tested nanoparticles and set aside for 24 h (37°C/5% $CO_2$). After the lapse of the test time, the medium was removed and replaced with 50 $\mu$l of an MTT reagent solution at a concentration of 1 mg/ml. The plate was incubated for 2 hours (37°C/5% $CO_2$). Subsequently, the MTT solution was removed, and 100 $\mu$l of isopropanol was added. After shaking the plate on the shaker for 2 minutes, the results for 570 nm wavelength were read (reference value of 650 nm). The mean value of absorbance read OD570 of the cells not exposed to a toxic agent (control) should be above 0.7 [see literature item 206].

*Data Analysis*

A decrease in the number of viable cells causes a decrease in metabolic activity in the sample. This reduction directly correlates with the amount of blue and purple formazan formed, monitored basing on the optical density at 570 nm. The following equation (C.1) is used to calculate viability reduction compared to control:

$$V_{iab\,\%} = \frac{100 \times OD_{570\,e}}{OD_{570b}}$$

where: • OD570e is the mean value of the measured absorbance of the test samples; • OD570b is the mean value of the measured absorbance of the control.

The results obtained were statistically analysed using the Dixon Q test.

**Table 1.** Tested cell lines:

| Cell line (derived from tumour - tumour testing model) | Source | Medium used |
|---|---|---|
| **MCF-7** breast/mammary gland | ECACC | DMEM 10% foetal bovine serum 1% Penicillin-Streptomycin L-glutamine 2mM |
| **AU565** breast/mammary gland | ATCC® | RPMI-1640 10% foetal bovine serum 1% Penicillin-Streptomycin L-glutamine 2mM |
| **HeLa** uterine cervix | ECACC | DMEM 10% foetal bovine serum 1% Penicillin-Streptomycin L-glutamine 2mM |
| **PANC-1** pancreas | ECACC | DMEM 10% foetal bovine serum 1% Penicillin-Streptomycin L-glutamine 2mM |
| **OVCAR-3** Ovary | ATCC® | RPMI-1640 20% foetal bovine serum 1% Penicillin-Streptomycin L-glutamine 2 mM + insulin |
| **ACHN** Kidneys | ECACC | DMEM 10% foetal bovine serum 1% Penicillin-Streptomycin L-glutamine 2mM |
| **A2780** Ovary | ECACC | RPMI-1640 10% foetal bovine serum 1% Penicillin-Streptomycin L-glutamine 2mM |
| **A549** Lung | ECACC | DMEM 10% foetal bovine serum 1% Penicillin-Streptomycin L-glutamine 2mM |
| **COLO 205** Large intestine/colon | ECACC | RPMI-1640 10% foetal bovine serum 1% Penicillin-Streptomycin L-glutamine 2mM |

The toxicity of the test nanoparticle formulation containing an anthracycline should be similar to that of a pure drug. There should be no toxicity of nanoparticles as such.

*Toxicity analysis of polysaccharide nanoparticles without anthracycline*

The obtained results confirmed the lack of toxicity of the carrier alone in the form of polysaccharide nanoparticles in the form of dextran nanoparticles for all cell lines tested. Similar results were obtained for the other polysaccharide carriers (carriers in the form of nanoparticles of cellulose, amylose, starch and heparin).
The highest concentration of nanoparticles (2.5 mg nanoparticles/ml) causes up to 20% decrease in toxicity **(Table 1**).

Table 1. Shows the result of the MTT cytotoxicity test on the HeLa cell line for the dextran carrier

| NPs-carrier | | | | | | | | |
|---|---|---|---|---|---|---|---|---|
| Concentration of NPs [ng/ml] | 1.00E+07 | 5.00E+06 | 2.50E+06 | 2.50E+05 | 2.50E+04 | 2.50E+03 | 2.50E+02 | 2.50E+01 |
| Average cell viability [%] | 75.437 | 82.389 | 87.647 | 91.003 | 107.418 | 104.579 | 106.158 | 104.212 |
| SD [%] | 0.978 | 0.223 | 0.104 | 1.969 | 2.929 | 1.196 | 3.251 | 3.658 |

where SD: standard deviation for n=8

*Toxicity analysis of polysaccharide nanoparticles with anthracycline*

Studies conducted for anthracycline-containing nanoparticles (produced according to Example 1) mostly showed a toxicity profile similar to that of a pure drug (e.g. **Fig. 2,** toxicity graph of DAU nanoparticles for the MCF-7 cell line and EPI nanoparticles for the COLO 205 cell line). On the other hand, higher *in vitro* toxicity was observed for some cell lines despite the use of an equimolar drug concentration (e.g. **Fig. 3 D** (OVCAR3_NPs-DAU) toxicity graph for DAU-dextran nanoparticles on the OVCAR-3 cell line, **Fig. 2 A, B, C, F, G** toxicity graphs for NPs_EPI). Such a result indicates that selected cell lines, and, consequently, the corresponding tumours, are more sensitive to drugs encapsulated in polysaccharide nanoparticles due to no defence mechanisms against drugs encapsulated in polysaccharide nanoparticles or increased uptake of such structures by cells. For the animal tests disclosed in the following examples, tumour lines were selected with a higher toxic response on a given tumour line than the pure drug or which had at least the same response. Such a choice was dictated by the expected higher efficacy of the formulation against tumour or at least the same efficacy of the formulation in the form encapsulated with the polysaccharide. The obtained results are presented in the graphs in **Fig. 2** and **Fig. 3** and in **Table 2** below.

Table 2. shows the results of MTT NPs_Epi and NPs_Dau cytotoxicity test versus Epi and Dau for some tumour lines. Where: NPs_Epi/NPs_Dau: epirubicin/daunorubicin encapsulated with dextran nanoparticles; Epi, Dau is epirubicin and daunorubicin administered as a pure non-encapsulated drug, respectively; SD: is standard deviation expressed in % for 8 test replicates for a specific drug concentration or NPs-drug concentration

| A2780_Epi | | | | | | | | | |
|---|---|---|---|---|---|---|---|---|---|
| Concentration of NPs [ng/ml] | 5.00E+03 | 1.25E+04 | 2.50E+04 | 5.00E+04 | 1.2E+05 | 2.50E+05 | 5.00E+05 | 1.25E+06 | 2.50E+06 |
| Average viability [%] | 95.203 | 90.784 | 89.24 | 83.629 | 69.996 | 58.252 | 41.752 | 14.869 | 3.102 |
| SD [%] | 4.656 | 1.756 | 5.715 | 6.047 | 4.455 | 8.266 | 6.192 | 2.8 | 0.434 |
| | | | | | | | | | |
| A2780_NPs_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 87.857 | 85.783 | 87.666 | 88.661 | 71.576 | 56.884 | 16.563 | 1.432 | 1.38 |
| SD [%] | 2.64 | 2.35 | 2.76 | 3.189 | 3.084 | 2.744 | 2 | 0.615 | 0.511 |
| | | | | | | | | | |
| A549_NPs_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 103.66 | 96.938 | 96.926 | 93.941 | 82.055 | 71.201 | 48.475 | 2.267 | 2.565 |
| SD [%] | 6.278 | 6.107 | 5.521 | 4.269 | 5.202 | 11.099 | 7.152 | 0.443 | 0.834 |
| | | | | | | | | | |
| A549_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 103.44 | 95.631 | 88.608 | 74.693 | 83.913 | 75.153 | 62.796 | 29.508 | 23.423 |
| SD [%] | 3.412 | 2.589 | 6.123 | 10.105 | 4.674 | 10.91 | 12.273 | 7.877 | 5.337 |
| | | | | | | | | | |
| ACHN_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 81.178 | 65.301 | 63.713 | 59.828 | 63.237 | 60.112 | 50.916 | 46.66 | 32.352 |
| SD [%] | 7.627 | 3.938 | 3.629 | 3.093 | 3.669 | 2.166 | 2.569 | 2.795 | 3.797 |
| | | | | | | | | | |

(continued)

| ACHN_NPs_Epi | | | | | | | | | |
|---|---|---|---|---|---|---|---|---|---|
| Concentration of NPs [ng/ml] | 5000 | 12500 | 25000 | 50000 | 125000 | 250000 | 500000 | 1250000 | 2500000 |
| Average viability [%] | 89.542 | 73.785 | 69.871 | 66.614 | 62.764 | 62.586 | 46.799 | 2.038 | 1.872 |
| SD[%] | 5.396 | 5.251 | 5.281 | 2.844 | 6.691 | 3.261 | 2.731 | 0.98 | 0.398 |
| | | | | | | | | | |
| AU565_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 84.738 | 66.48 | 66.439 | 48.052 | 48.38 | 29.222 | 11.723 | 1.912 | 1.822 |
| SD [%] | 5.662 | 2.336 | 4.299 | 4.205 | 3.48 | 2.693 | 0.87 | 0.347 | 0.517 |
| | | | | | | | | | |
| AU565_NPs_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 89.859 | 78.632 | 73.084 | 64.315 | 52.404 | 44.84 | 28.783 | 1.501 | 1.484 |
| SD [%] | 4.847 | 5.117 | 3.234 | 3.906 | 3.999 | 5.056 | 4.97 | 0.864 | 0.734 |
| | | | | | | | | | |
| Colo205_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 83.936 | 63.431 | 66.212 | 61.427 | 55.748 | 49.549 | 23.946 | 5.063 | 2.239 |
| SD [%] | 2.771 | 5.072 | 3.61 | 6.188 | 3.73 | 4.79 | 5.206 | 1.233 | 1.137 |
| | | | | | | | | | |
| Colo205_NPs_Epi | | | | | | | | | |
| Concentration of NPs [ng/ml] | 5.00E+03 | 1.25E+04 | 25000 | 5.00E+04 | 1.25E+05 | 2.50E+05 | 5.00E+05 | 1.25E+06 | 2.50E+06 |
| Average viability [%] | 111.719 | 102.72 | 87.182 | 62.646 | 66.796 | 53.903 | 26.718 | 2.136 | 1.231 |
| SD [%] | 10.617 | 3.581 | 7.267 | 3.398 | 6.308 | 6.918 | 3.188 | 1.817 | 0.31 |
| | | | | | | | | | |
| HeLa_Epi | | | | | | | | | |
| Concentration of NPs [ng/ml] | 5.00E+03 | 1.25E+04 | 25000 | 5.00E+04 | 1.25E+05 | 2.50E+05 | 5.00E+05 | 1.25E+06 | 2.50E+06 |

| HeLa_Epi | | | | | | | | | |
|---|---|---|---|---|---|---|---|---|---|
| Average viability [%] | 100.18 | 98.38 | 72.85 | 17.04 | 12.73 | 14.93 | 7.96 | 3.6 | 4.21 |
| SD [%] | 9.75 | 10.77 | 3.11 | 2.13 | 1.42 | 1.86 | 1.77 | 0.79 | 0.42 |
| | | | | | | | | | |
| HeLa_NPs_Epi | | | | | | | | | |
| Concentration of NPs [ng/ml] | 5.00E+03 | 1.25E+04 | 25000 | 5.00E+04 | 1.25E+05 | 2.50E+05 | 5.00E+05 | 1.25E+06 | 2.50E+06 |
| Average viability [%] | 105.72 | 105.61 | 97.67 | 42.81 | 21.77 | 12.78 | 1.64 | 2.4 | 1.84 |
| SD [%] | 3.72 | 1.66 | 5.31 | 2.03 | 1.23 | 0.41 | 0.75 | 1 | 0.33 |
| | | | | | | | | | |
| MCF-7_Epi | | | | | | | | | |
| Concentration of NPs [ng/ml] | 5.00E+03 | 1.25E+04 | 25000 | 5.00E+04 | 1.25E+05 | 2.50E+05 | 5.00E+05 | 1.25E+06 | 2.50E+06 |
| Average viability [%] | 97.78 | 88.51 | 64.7 | 56.01 | 46.97 | 41.35 | 31.11 | 5.9 | 1.68 |
| SD [%] | 1.57 | 6.29 | 3.44 | 2.53 | 2.12 | 2.37 | 4.22 | 0.59 | 0.45 |
| | | | | | | | | | |
| MCF-7_NPs_Epi | | | | | | | | | |
| Concentration of NPs [ng/ml] | 5.00E+03 | 1.25E+04 | 25000 | 5.00E+04 | 1.25E+05 | 2.50E+05 | 5.00E+05 | 1.25E+06 | 2.50E+06 |
| Average viability [%] | 101.89 | 91.3607 | 80.0486 | 55.3861 | 54.0362 | 47.8267 | 8.61231 | 1.13391 | 1.12041 |
| SD [%] | 6.48 | 8.73 | 7.26 | 5.16 | 5.53 | 7.29 | 1.54 | 0.15 | 0.31 |
| | | | | | | | | | |
| OVCAR3_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 104.596 | 102.184 | 99.208 | 92.151 | 91.668 | 88.222 | 75.986 | 58.803 | 33.266 |
| SD [%] | 11.436 | 8.525 | 9.347 | 8.336 | 7.569 | 7.035 | 6.199 | 7.362 | 1.856 |
| | | | | | | | | | |
| OVCAR3_NPs_Epi | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 104.928 | 103.836 | 104.979 | 100.735 | 87.78 | 76.81 | 51.264 | 8.307 | 1.575 |

(continued)

| | | | | | | | | | |
|---|---|---|---|---|---|---|---|---|---|
| **OVCAR3_NPs_Epi** | | | | | | | | | |
| SD [%] | 0.428 | 4.354 | 3.897 | 3.517 | 4.896 | 3.37 | 5.685 | 7.684 | 5.028 |
| **PANC1_Epi** | | | | | | | | | |
| Concentration of NPs [ng/ml] | 2.50E+06 | 1.25E+06 | 5.00E+05 | 2.50E+05 | 1.25E+05 | 5.00E+04 | 2.50E+04 | 1.25E+04 | 5.00E+03 |
| Average viability [%] | 5.339 | 27.965 | 54.929 | 79.499 | 82.872 | 89.898 | 99.155 | 94.813 | 97.954 |
| SD [%] | 1.299 | 5.771 | 6.351 | 6.948 | 7.933 | 10.598 | 9.519 | 8.039 | 8.867 |
| **PANC1_NPs_Epi** | | | | | | | | | |
| Concentration of NPs [ng/ml] | 2.50E+06 | 1.25E+06 | 5.00E+05 | 2.50E+05 | 1.25E+05 | 5.00E+04 | 2.50E+04 | 1.25E+04 | 5.00E+03 |
| Average viability [%] | 1.777 | 1.909 | 45.271 | 81.874 | 86.271 | 94.913 | 97.858 | 93.654 | 95.844 |
| SD [%] | 0.284 | 0.555 | 3.47 | 3.87 | 4.051 | 3.017 | 7.306 | 5.859 | 3.811 |
| **A2780_Dau** | | | | | | | | | |
| Concentration of NPs [ng/ml] | 2.50E+06 | 1.25E+06 | 5.00E+05 | 2.50E+05 | 1.25E+05 | 5.00E+04 | 2.50E+04 | 1.25E+04 | 5.00E+03 |
| Average viability [%] | 54.259 | 60.422 | 60.621 | 35.542 | 18.312 | 11.600 | 3.689 | 0.540 | 0.295 |
| SD [%] | 4.656 | 8.988 | 4.390 | 5.641 | 2.355 | 1.770 | 0.931 | 0.223 | 0.035 |
| **A2780_NPs_Dau** | | | | | | | | | |
| Concentration of NPs [ng/ml] | 2.50E+06 | 1.25E+06 | 5.00E+05 | 2.50E+05 | 1.25E+05 | 5.00E+04 | 2.50E+04 | 1.25E+04 | 5.00E+03 |
| Average viability [%] | 43.611 | 40.073 | 42.996 | 28.381 | 22.915 | 14.843 | 1.150 | 0.574 | 1.514 |
| SD [%] | 12.369 | 14.150 | 8.623 | 6.746 | 4.256 | 1.073 | 0.346 | 0.087 | 0.937 |
| **AU565_Dau** | | | | | | | | | |
| Concentration of NPs [ng/ml] | 2.50E+06 | 1.25E+06 | 5.00E+05 | 2.50E+05 | 1.25E+05 | 5.00E+04 | 2.50E+04 | 1.25E+04 | 5.00E+03 |
| Average viability [%] | 1.052948 | 1.544017 | 3.244951 | 12.13614 | 25.83484 | 28.25821 | 33.21763 | 58.57172 | 75.95658 |
| SD [%] | 0.760732 | 0.521341 | 0.366567 | 0.876678 | 2.585976 | 1.90153 | 2.721307 | 3.629251 | 2.539451 |

| AU565_Dau | | | | | | | | | |
|---|---|---|---|---|---|---|---|---|---|
| | | | | | | | | | |
| **AU565_NPs_Dau** | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 71.95658 | 52.57172 | 32.21763 | 29.25821 | 30.83484 | 11.13614 | 2.244951 | 1.344017 | 2.052948 |
| SD [%] | 2.539451 | 3.629251 | 2.721307 | 1.90153 | 2.585976 | 0.876678 | 0.366567 | 0.521341 | 0.760732 |
| | | | | | | | | | |
| **HeLa_Dau** | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 2.054 | 2.851 | 2.108 | 4.138 | 7.890 | 7.579 | 28.287 | 107.342 | 111.366 |
| SD [%] | 0.592 | 1.899 | 1.524 | 0.857 | 2.888 | 0.962 | 2.600 | 3.105 | 6.636 |
| | | | | | | | | | |
| **HeLa_NPs_Dau** | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 3.324 | 3.726 | 4.734 | 6.759 | 5.897 | 13.776 | 34.299 | 83.013 | 98.005 |
| SD [%] | 1.583 | 2.104 | 2.407 | 0.827 | 1.122 | 2.282 | 4.639 | 9.485 | 2.638 |
| | | | | | | | | | |
| **MCF-7_Dau** | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 1.311 | 1.153 | 1.072 | 4.421 | 17.162 | 23.086 | 25.325 | 40.867 | 69.450 |
| SD [%] | 0.625 | 0.312 | 0.371 | 2.079 | 6.357 | 6.812 | 5.820 | 4.646 | 3.975 |
| | | | | | | | | | |
| **MCF-7_NPs_Dau** | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 1.585 | 2.972 | 1.132 | 11.281 | 19.679 | 20.980 | 20.504 | 29.155 | 61.507 |
| SD [%] | 0.266 | 1.500 | 0.224 | 3.806 | 3.086 | 3.062 | 3.368 | 4.225 | 8.928 |
| | | | | | | | | | |

**OVCAR-3_Dau**

| | | | | | | | | | |
|---|---|---|---|---|---|---|---|---|---|
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 1.330 | 3.616 | 43.386 | 52.550 | 59.635 | 71.147 | 120.518 | 94.253 | 99.822 |
| SD [%] | 0.251 | 2.964 | 1.967 | 3.236 | 2.514 | 5.825 | 8.619 | 8.962 | 7.221 |

**OVCAR-3_NPs_Dau**

| | | | | | | | | | |
|---|---|---|---|---|---|---|---|---|---|
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 3.400 | 2.580 | 31.840 | 50.660 | 50.740 | 60.560 | 83.543 | 82.286 | 75.566 |
| SD [%] | 1.245 | 0.723 | 2.189 | 2.464 | 2.535 | 2.852 | 6.593 | 12.896 | 7.598 |

**Colo205_Dau**

| | | | | | | | | | |
|---|---|---|---|---|---|---|---|---|---|
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 30.89 | 34.20 | 19.17 | 4.46 | 3.75 | 3.47 | 2.93 | 5.07 | 30.89 |
| SD [%] | 3.63 | 1.77 | 1.43 | 2.96 | 1.88 | 1.93 | 0.93 | 1.44 | 3.63 |

**Colo205_NPs_Dau**

| | | | | | | | | | |
|---|---|---|---|---|---|---|---|---|---|
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 85.11 | 58.04 | 38.61 | 26.77 | 30.98 | 19.95 | 3.06 | 2.82 | 85.11 |
| SD [%] | 4.61 | 4.39 | 1.73 | 1.32 | 2.10 | 2.48 | 0.26 | 0.28 | 4.61 |

**PANC-1_Dau**

| | | | | | | | | | |
|---|---|---|---|---|---|---|---|---|---|
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 95.12 | 93.89 | 61.24 | 56.67 | 48.79 | 33.21 | 0.54 | 1.02 | 95.12 |
| SD [%] | 5.16 | 4.73 | 3.88 | 3.67 | 2.83 | 3.51 | 0.09 | 0.48 | 5.16 |

**PANC-1_NPs_Dau**

| | | | | | | | | | |
|---|---|---|---|---|---|---|---|---|---|
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |

| PANC-1_NPs_Dau | | | | | | | | | |
|---|---|---|---|---|---|---|---|---|---|
| Average viability [%] | 94.94 | 90.75 | 70.25 | 59.95 | 51.99 | 54.42 | 23.38 | 2.76 | 94.94 |
| SD [%] | 6.91 | 1.78 | 4.67 | 4.79 | 3.07 | 3.38 | 3.77 | 0.94 | 6.91 |
| | | | | | | | | | |
| ACHN_Dau | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 62.65 | 43.86 | 1.60 | 2.88 | 2.75 | 2.24 | 3.02 | 5.35 | 62.65 |
| SD [%] | 12.06 | 6.85 | 0.45 | 2.02 | 1.81 | 0.67 | 0.58 | 0.68 | 12.06 |
| | | | | | | | | | |
| ACHN_NPs_Dau | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 66.26 | 71.36 | 65.71 | 74.82 | 75.62 | 22.85 | 1.12 | 1.23 | 66.26 |
| SD [%] | 10.10 | 7.85 | 6.07 | 7.31 | 8.19 | 5.58 | 0.28 | 0.11 | 10.10 |
| | | | | | | | | | |
| A549_Dau | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 65.72 | 57.00 | 55.31 | 50.80 | 49.55 | 52.82 | 0.60 | 0.53 | 65.72 |
| SD [%] | 8.49 | 8.01 | 4.37 | 2.30 | 1.16 | 1.80 | 0.09 | 0.09 | 8.49 |
| | | | | | | | | | |
| A549_NPs_Dau | | | | | | | | | |
| Concentration of NPs [ng/ml] | 5.00E+03 | 1.25E+04 | 2.50E+04 | 5.00E+04 | 1.25E+05 | 2.50E+05 | 5.00E+05 | 1.25E+06 | 2.50E+06 |
| Average viability [%] | 80.73 | 74.25 | 59.67 | 60.67 | 56.27 | 54.55 | 14.77 | 1.34 | 80.73 |
| SD [%] | 0.71 | 1.22 | 2.84 | 2.09 | 1.72 | 4.09 | 2.22 | 0.98 | 0.71 |

### Example 3.

**Determination of anti-tumour efficacy of anthracyclines encapsulated with a polysaccharide**

*A) Determination of a maximum tolerated dose (MTD) for dextran-encapsulated epirubicin (NPs-EPI)*

The evaluation of acute toxicity of anthracycline encapsulated with a polysaccharide in the form of dextran-encapsulated epirubicin (NPs-EPI) as produced in **Example 1** with MTD determination was performed using the **acute oral toxicity - up-and-down** procedure according to the OECD procedure no. 425 with a modification of the administration route of the test material.

Intravenous administration *(i.v.;* to the caudal vein) was dictated by how epirubicin in its currently used form, i.e. epirubicin hydrochloride (EPI), is administered to patients, and NPs-EPI will be administered if its anti-tumour efficacy is demonstrated.

The acute toxicity assessment method used is an alternative method recommended by the OECD (OECD procedure 425), which takes into account the aim to improve animal welfare and the 3Rs principle. *(Replacement, Reduction, Refinement).*

The higher-lower dose acute toxicity assessment procedure involves administering test material to an individual animal at a single dose lower than the expected median lethal dose ($LD_{50}$). Depending on the effect obtained following the administration of the first dose of the test or reference material, the next subject was administered a dose increased or reduced by a fixed coefficient. This procedure was continued sequentially until achieving a dose, the increase by which (by the established coefficient) caused death, and the decrease (by the established coefficient) resulted in the survival of the animal.

Following the determination of MTD for NPs-EPI (dextran-encapsulated epirubicin) in part A of the experiment, part B of the experiment was conducted to compare the acute toxicity and MTD of free EPI (in the form of epirubicin hydrochloride) at a dose equivalent to the dose of the drug contained in the combination thereof with NPs (EPI-NPs) at a dose constituting the MTD.

*B) Comparative assessment of acute toxicity and MTD of free EPI at a dose equivalent to that of the drug contained in the combination thereof with NPs (NPs-EPI) at a dose constituting the MTD*

Acute toxicity assessment of EPI at a dose equivalent to its content in the dose constituting the MTD of NPs-EPI (determined during the implementation of part A of the experiment) was carried out using the up-and-down method according to OECD procedure no. 425.

Dextran-encapsulated epirubicin (NPs-EPI) and epirubicin in free form, i.e. of epirubicin hydrochloride EPI were administered to mice once, to the caudal vein. Two hours before the administration of NPs-EPI and EPI, the animals were deprived of food. 30 minutes after the administration of NPs-EPI (test material) and EPI (reference material), the feed was again made available to the mice. The administration of another dose to the next animal took place after the result of the previous dose administration was obtained. The baseline dose of NPs-EPI contained the equivalent of 27.39 mg EPI/kg bw (body weight) The EPI as reference material was administered at a dose of 30 and 31 mg/kg bw NPs-EPI and EPI were administered to mice in the form of aqueous solutions (water for injections) in volumes of no more than 0.18 cm$^3$/mouse. The amount of the dose administered to the next animal depended on the result of administration of the previous dose. If the animal survived 48 hours after administration, the dose for the next animal was increased by a modified coefficient of more than 1 and less than 1.3 (coefficients 1.02; 1.05; 1.10 and 1.15 were used). If the animal died, the dose for the next subject was reduced by the same coefficient. The test material was administered until the dose was reached where 3 subsequent animals survived the administration of the highest dose. By OECD procedure no. 425, the recommended dose modification coefficient is 3.2, but due to the small difference between the minimum therapeutic dose and the MTD for the EPI, it was necessary to use a coefficient by which successive doses of the new formulation of said drug (NPs-EPI) were modified which was significantly less than 1.3 (not as provided for in the OECD 425). The method of individual coding animals was not known to persons who took care of the animals and performed the planned procedures being part of the study.

Accordingly, the maximum tolerated dose (MTD) for anthracycline NPs-EPI encapsulated with a polysaccharide for intravenous administration to mice was determined at 30 mg/kg bw (based on EPI) (90 mg/m$^2$).

*C) Determination of the anti-tumour efficacy of epirubicin encapsulated in dextran nanoparticles in ovarian tumour*

The subject of the study is to determine the anti-tumour efficacy and potential adverse effects of NPs-EPI therapy versus the classical form of EPI in mice with implanted xenograft (ovarian tumour model). The study used epirubicin encapsulated in dextran nanoparticles (NPs-EPI) prepared according to **Example 1.** Epirubicin does not penetrate the blood-brain barrier, and it is eliminated in three stages. The biological half-life ($t_{1/2}$) is 15 - 45 h, approx. 40 h on average.

### Obtaining a tumour study model

To develop a tumour model, female mice from the CByJ.Cg-Foxn1<nu>/cmdb inbred strain were implanted OVCAR3 line ovarian tumour cells. Following anaesthesia, $5 \times 10^5$ ovarian tumour cells (OVCAR3 line cells) in the volume of 100 μl of PBS and Matrigel were subcutaneous (s.c.) injected into the right side of the female mice. During cell implantation, the animals did not experience any pain, as they were previously introduced into a state of mild inhalation narcosis using isoflurane.

After the tumour grew to the size of approx. 150 mm$^3$, the mice were divided into 3 groups:

K1/OVCAR3 - control group **A,** which received water for injections;
NPs/EPI/OVCAR3 - the group which received epirubicin encapsulated with dextran nanoparticles (NPs-EPI) in the amount of 22.5 mg/kg bw (67.5 mg/m$^2$) - test group **B,** and test group **C** which received NPs-EPI in the amount of 3.75 mg/kg bw (11.25 mg/m$^2$);
EPI/OVCAR3 - reference group **D,** which received epirubicin in the standard form as epirubicin hydrochloride in the amount of 15 mg/kg bw (45 mg/m$^2$).

All mice, except for the animals constituting the reference group, were treated with EPI encapsulated in dextran nanoparticles (NPs-EPI) or the standard form (EPI). During the treatment, the size of the tumours was measured, and the animals were closely observed. After the end of treatment, tumour measurements, haematological examinations, as well as macroscopic and histopathological examinations of internal organs and tumours were performed to allow the assessment of the efficacy and safety of using the NPs-EPI anti-tumour therapy in comparison to the standard form of the drug.

### Procedures performed on the K1/OVCAR3 control group (administration of water for injection into the caudal vein)

Females with induced tumours, constituting the reference group (K1/OVCAR3), qualified for the anti-tumour efficacy assessment of NPs-EPI in a mouse model of ovarian tumour (OVCAR3 cell line), were administered water for injection every second day (10 administrations) into the caudal vein. Water for injection was administered (duration of single administration - 30 seconds/mouse) at a volume of 0.18 cm$^3$/mouse. During intravenous administration of water for injection, the animals did not experience any pain associated with the method of administration as the site of administration had previously been anaesthetised by spraying it with 10% lidocaine. The water for injection was administered 10 times. All mice in this group were sacrificed one day after the 10$^{th}$ administration of water for injection.

### Procedure in the EPI/OVCAR3 test group - mouse model of the ovarian tumour with implanted OVCAR3 line cells

Female mice with induced tumours qualified for EPI/OVCAR3 anti-tumour efficacy assessment in a mouse model of ovarian tumour - the OVCAR3 cell line, EPI was administered into the caudal vein (application duration - 30 seconds/mouse), once daily, every second day (2 administrations) at a dose equivalent to the drug content in a dose of 15 mg/kg bw EPI (1/2 MTD for epirubicin in dextran nanoparticles) was administered to mice in the form of aqueous solutions in volumes of no more than 0.18 cm$^3$/mouse. To administer the same dose of EPI to the animals, the animals were weighed before each administration, and the volume of the solutions administered was modified as a function of the change in body weight. During intravenous administration of EPI solutions, the animals did not experience any pain associated with the method of administration as the site of administration was anaesthetised by spraying it with 10% lidocaine.

### Administration of NPs-EPI in the NPsEPI/OVCAR3 test group at doses of 3.75 and 22.5 mg/kg bw in a mouse model of the ovarian tumour with implanted OVCAR3 line cells

Female mice with induced tumours qualified for NPs-EPI anti-tumour efficacy assessment in the mouse model of ovarian tumour (OVCAR3 cell line) were administered NPs-EPI into the caudal vein once a day, every second day at doses of 3.75 mg/kg bw constituting 1/8, and 22.5 mg/kg bw constituting 3/4 of the MTD for NPs-EPI administered to mice as aqueous solutions in volumes of no more than 0.18 cm$^3$/mouse.

To administer the same dose of NPs-EPI to the animals, the animals were weighed before each administration, and the volume of the solutions administered was modified as a function of the change in body weight. During intravenous administration of NPs-EPI solutions, the animals did not experience any pain associated with the method of administration as the site of administration had previously been anaesthetised by spraying it with 10% lidocaine.

The obtained results are presented in graphs in **Fig. 4 A** and **B** and in the **Tables 4-15** below.

**Table 4.** Body weight of the mice in the K1/OVCAR group during intravenous administration of water for injection (detailed results).

| Group | Mouse # | Body weight (g) before the subsequent administration/necropsy | | | | | | | | | | | Weight gain (g) |
|---|---|---|---|---|---|---|---|---|---|---|---|---|---|
| | | Before 1 | Before 2 | Before 3 | Before 4 | Before 5 | Before 6 | Before 7 | Before 8 | Before 9 | Before 10 | Before necropsy | |
| Injection water (group A) | 3 | 23.30 | 23.90 | 24.10 | 23.90 | 24.20 | 24.00 | 24.65 | 24.00 | 24.30 | 24.30 | 23.50 | 0.20 |
| | 7 | 24.60 | 25.20 | 25.00 | 25.50 | 25.40 | 25.20 | 25.60 | 25.30 | 25.80 | 25.10 | 25.30 | 0.70 |
| | 14 | 23.00 | 23.60 | 23.50 | 23.50 | 23.30 | 23.00 | 23.75 | 23.10 | 23.75 | 23.50 | 23.40 | 0.40 |
| | 28 | 21.40 | 21.90 | 22.20 | 22.20 | 21.90 | 21.60 | 22.15 | 21.50 | 22.10 | 22.00 | 21.50 | 0.10 |
| | 30 | 24.20 | 25.10 | 25.60 | 25.40 | 25.20 | 25.40 | 25.40 | 25.60 | 25.85 | 25.70 | 26.00 | 1.80 |
| | 33 | 23.50 | 23.50 | 23.30 | 23.60 | 23.70 | 23.30 | 23.80 | 23.30 | 24.10 | 24.40 | 23.80 | 0.30 |
| Average ± SD | | 23.33 ±1.12 | 23.87 ±1.21 | 23.95 ±1.23 | 24.02 ±1.25 | 23.95 ±1.30 | 23.75 ±1.43 | 24.23 ±1.28 | 23.80 ±1.52 | 24.32 ±1.40 | 24.17 ±1.30 | 23.92 ±1.58 | 0.58 ±0.63 |

**Table 5.** Tumour size in mice in the K1/OVCAR group during intravenous administration of water for injection (detailed results)

| Group | Mouse # | Tumour volume (mm³) before subsequent administration/necropsy | | | | | | | | | | | Change in tumour volume (mm3) | Volume of the tumour following dissection (mm3) |
|---|---|---|---|---|---|---|---|---|---|---|---|---|---|---|
| | | Before 1 | Before 2 | Before 3 | Before 4 | Before 5 | Before 6 | Before 7 | Before 8 | Before 9 | Before 10 | Before necropsy | | |
| Injection water (group A) | 3 | 165.14 | 174.13 | 165.01 | 166.79 | 153.79 | 145.27 | 137.96 | 131.00 | 172.48 | 175.32 | 136.32 | -28.82 | 52.99 |
| | 7 | 201.10 | 224.27 | 295.36 | 239.06 | 255.51 | 263.93 | 301.04 | 311.46 | 335.91 | 271.05 | 263.97 | 62.87 | 141.53 |
| | 14 | 189.04 | 187.88 | 273.96 | 270.54 | 287.88 | 287.02 | 331.26 | 322.54 | 415.94 | 331.26 | 356.99 | 167.95 | 174.53 |
| | 28 | 197.47 | 182.12 | 205.59 | 164.67 | 174.14 | 140.61 | 156.86 | 154.08 | 149.65 | 147.70 | 126.88 | -70.59 | 58.98 |
| | 30 | 192.79 | 178.29 | 218.98 | 200.23 | 205.02 | 164.36 | 180.20 | 172.12 | 192.30 | 206.04 | 209.76 | 16.97 | 82.07 |
| | 33 | 219.39 | 277.07 | 325.78 | 230.79 | 296.25 | 261.86 | 370.74 | 369.15 | 355.27 | 400.12 | 465.61 | 246.22 | 297.65 |
| Average ± SD | | 194.16 ±17.70 | 203.96 ±40.11 | 247.45 ±60.82 | 212.01 ±42.27 | 228.77 ±59.89 | 210.51 ±67.26 | 246.34 ±99.81 | 243.39± 102.37 | 270.26 ± 112.20 | 255.25 ±97.36 | 259.92 ±132.09 | | 134.63 ± 93.13 |

Table 6. Results of macroscopic observations during the necropsy of the mice in the K1/OVCAR group, which was administered water for injection (mouse no. 3)

| External appearance of the mouse | Skin thickness 0.30 mm |
|---|---|
| **Organs** | **Macroscopic evaluation of internal organs** |
| Liver | No visible changes |
| Left kidney | No visible changes |
| Right kidney | No visible changes |
| Adrenal glands | No visible changes |
| Spleen | No visible changes |
| Pancreas | No visible changes |
| Lung | No visible changes |
| Heart | No visible changes |
| Thymus | No visible changes |

(continued)

| External appearance of the mouse | Skin thickness 0.30 mm |
|---|---|
| **Organs** | **Macroscopic evaluation of internal organs** |
| Intestines | Intestines filled with green food content, the content prevents an accurate assessment of the mucous membrane |
| Stomach | No visible changes |
| Reproductive organs | No visible changes |
| Bladder | No visible changes |
| Brain | No visible changes |
| Tumour | 5.40 mm x 4.43 mm in size in the subcutaneous tissue, immobile about the skin. |

**Table 7.** Body weight of the mice from the NPsEPI/OVCAR3 group during intravenous administration of NPs-EPI at a dose of 3.75 mg/kg bw (detailed results)

| Group | Mouse # | Weight (g) | | | | | Weight loss (g) |
|---|---|---|---|---|---|---|---|
| | | Before 1st administration | Before 2nd administration | Before 3rd administration | Before 4th administration | Before necropsy | |
| EP-NPs 3 3.75 mg/kg bw (group C) | 17 | 23.00 | 22.80 | 22.50 | 20.50 | 19.00 | 4,00 |
| | 18 | 23.50 | 22.60 | 22.20 | 20.10 | 18.40 | 5,10 |
| | 19 | 23.50 | 22.60 | 22.60 | 21.40 | 19.60 | 3,90 |
| | 20 | 24.20 | 23.30 | 23.10 | 21.50 | 19.10 | 5,10 |
| | 22 | 22.60 | 22.10 | 21.80 | 20.80 | 18.00 | 4,60 |
| | 34 | 25.00 | 24.50 | 23.30 | 22.85 | 21.40 | 3,60 |
| | 38 | 20.50 | 20.30 | 20.40 | 19.15 | 17.00 | 3,50 |
| Average ± SD | | 23.19±1.42 | 22.60±1.27 | 22.27±0.97 | 20.90±1.18 | 18.93±1.38 | 4.26±0.68 |

**Table 8.** Tumour size in the mice from the NPsEPI/OVCAR3 group during intravenous administration of NPs-EPI at a dose of 3.75 mg/kg bw (detailed results).

| Group | Mouse # | Tumour volume (mm3) before/after subsequent administration | | | | | Change in tumour volume (mm3) | Percentage change in tumour volume (%) | Volume of the tumour following dissection (mm³) |
|---|---|---|---|---|---|---|---|---|---|
| | | Before 1st administration | Before 2nd administration | Before 3rd administration | Before 4th administration | One day after 4th administration | | | |
| EP-NPs in dose 3.75 mg/kg bw | 17 | 138.40 | 110.88 | 107.01 | 100.32 | 93.86 | -44.54 | 32.18 | 84.79 |
| | 18 | 162.50 | 160.99 | 150.13 | 145.76 | 143.01 | -19.49 | 11.99 | 101.79 |
| | 19 | 177.77 | 172.05 | 161.94 | 153.15 | 150.83 | -26.94 | 15.15 | 156.31 |
| | 20 | 126.90 | 108.02 | 98.20 | 83.33 | 59.70 | -67.20 | 52.95 | 71.65 |
| | 22 | 154.70 | 150.93 | 144.87 | 140.82 | 129.58 | -25.12 | 16.24 | 123.02 |
| | 34 | 153.42 | 139.71 | 129.42 | 124.97 | 108.87 | -44.55 | 29.04 | 95.78 |
| | 38 | 128.72 | 150.08 | 140.72 | 122.30 | 109.35 | -19.37 | 15.05 | 95.33 |
| Average ± SD | | 148.92 ±18.59 | 141.81 ± 24.28 | 133.18 ± 23.20 | 124.38 ±25.24 | 113.60 ± 1.20 | -25.54 ± 13.31 | 24.66 | 104.10 ± 27.87 |

**Table 9.** Results of macroscopic observations during necropsy of the NPsEPI/OVCAR3 group that received NPs-EPI at a dose of 3.75 mg/kg bw (mouse #17).

| External appearance of the mouse | Anal area dirty with faeces, mouse is cachectic. skin 0.30mm thick |
|---|---|
| **Organs** | **Macroscopic evaluation of internal organs** |
| Liver | No visible changes |
| Left kidney | No visible changes |
| Right kidney | No visible changes |
| Adrenal glands | No visible changes |
| Spleen | 1.5 cm x 0.4 cm x 0.2 cm in size (probably reduced) |
| Pancreas | No visible changes |
| Lung | No visible changes |
| Heart | No visible changes |
| Thymus | No visible changes |
| Intestines | Large intestine filled with liquid food content. the content prevents an accurate assessment of the mucous membrane |
| Stomach | No visible changes |
| Reproductive organs | No visible changes |
| Bladder | No visible changes |
| Brain | No visible changes |
| Tumour | 7.58 mm x 4.73 mm in size in the subcutaneous tissue, immobile about the skin. |

**Table 10. Body weight of the mice from the NPsEPI/OVCAR3 group during intravenous administration of NPs-EPI at a dose of 22.50 mg/kg bw (detailed results).**

| Group | Mouse # | Weight (g) | | | | | Weight loss (g) |
|---|---|---|---|---|---|---|---|
| | | Before 1st administration | Before 2nd administration | Before 3rd administration | Two days after 3rd administration | Before necropsy | |
| EP-NPs in dose 22.5 mg/kg bw (group B) | 4 | 24.90 | 23.40 | 22.90 | 20.20 | 18.61*** | 6.29 |
| | 10 | 24.50 | 23.10 | 21.40 | 19.40 | 17.50*** | 7.00 |
| | 13 | 23.20 | 21.80 | 20.10 | 17.60 | 15.90*** | 7.30 |
| | 21 | 24.50 | 23.30 | 22.20 | 21.40 | 19.60 | 4.90 |
| | 26 | 22.70 | 21.80 | 21.40 | 19.90 | 18.30*** | 4.40 |
| | 35 | 22.90 | 22.10 | 21.00 | 19.40 | 18.30 | 4.60 |
| | 37 | 23.80 | 23.60 | 21.50 | 18.80 | 17.30*** | 6.50 |
| | 39 | 21.20 | 20.70 | 18.30 | 15.30 | 14.40*** | 6.80 |
| Average ± SD | | 23.46±1.22 | 22.48±1.03 | 21.10±1.40 | 19.00±1.85 | 17.49±1.65 | 5.97±1.16 |

*** - weight of the mouse before the necropsy

**Table 11.** Tumour size in the mice from the NPsEPI/OVCAR3 group during intravenous administration of NPs-EPI at a dose of 22.5 mg/kg bw (detailed results).

| Group | Mouse # | Tumour volume (mm3) before/after subsequent administration | | | | | Change in tumour volume (mm³) | Percentage change in tumour volume (%) | Volume of the tumour following dissection (mm³) |
|---|---|---|---|---|---|---|---|---|---|
| | | Before 1st administration | Before 2nd administration | Before 3rd administration | One day after 3rd administration | Two day after 3rd administration | | | |
| Tumour volume (mm³) before/after subsequent | 4 | 154.67 | 198.95 | 112.71 | 61.82 | 91.30 | -63.37 | 40.97 | 44.72 |
| | 10 | 160.12 | 212.24 | 118.50 | 102.29 | 97.12 | -63.00 | 39.35 | 30.80 |
| | 13 | 155.36 | 225.70 | 134.31 | 105.40 | 105.32 | -50.04 | 32.21 | 83.40 |
| | 21 | 179.56 | 196.14 | 145.54 | 117.83 | 97.49 | -82.07 | 45.71 | 105.12 |
| | 26 | 152.73 | 184.59 | 112.33 | 104.39 | 96.39 | -56.34 | 36.89 | 49.30 |
| | 35 | 149.88 | 174.37 | 82.74 | 85.67 | 73.26 | -76.62 | 51.12 | 26.27 |
| | 37 | 176.02 | 188.07 | 156.66 | 114.05 | 132.35 | -43.67 | 24.81 | 59.37 |
| | 39 | 173.46 | 225.17 | 133.92 | 154.87 | 145.93 | -27.53 | 15.87 | 67.75 |
| Average ± SD | | 162.73 ±11.75 | 200.65 ± 18.87 | 124.59 ±23.08 | 105.79 ±26.66 | 104.90 ± 23.33 | -56.94 ±20.65 | 35.87 | 58.34 ± 26.62 |

**Table 12** Results of macroscopic observations during necropsy of the mouse no. 4 which received NPs-EPI at a dose of 22.5 mg/kg bw

| External appearance mice | Anal area dirty with faeces. mouse is cachectic |
|---|---|
| **Organs** | **Macroscopic evaluation of internal organs** |
| Liver | On the surface of the liver, there are many cream-colour changes, the size of a pinhead |
| Left kidney | No visible changes |
| Right kidney | No visible changes |
| Adrenal glands | No visible changes |
| Spleen | Dark cherry colour (slightly darker) |
| Pancreas | No visible changes |
| Lung | No visible changes |
| Heart | No visible changes |
| Thymus | No visible changes |
| Intestines | The intestines are filled with cream-like gastrointestinal contents. which content prevented an accurate assessment of the mucous membrane |
| Stomach | No visible changes |
| Reproductive organs | No visible changes |
| Bladder | No visible changes |
| Brain | No visible changes |
| Tumour | 6.16 mm x 3.81 mm in size in the subcutaneous tissue, immobile about the skin. |

**Table 13.** Body weight of the mice from the EPI/OVCAR3 group during intravenous administration of EPI at a dose of 15 mg/kg bw (detailed results).

| Group | Mouse # | Weight (g) | | | Weight loss (g) |
|---|---|---|---|---|---|
| | | Before 1st administration | Before 2nd administration | Before necropsy | |
| EP in a dose of 15 mg/kg bw (group D) | 5 | 23.80 | 23.65 | 20.55 | 3.25 |
| | 15 | 25.90 | 22.45 | 22.30 | 3.60 |
| | 24 | 23.80 | 23.10 | 22.25 | 1.55 |
| | 29 | 24.00 | 24.30 | 20.35 | 3.65 |
| | 31 | 23.10 | 22.45 | 20.60 | 2.50 |
| | 32 | 21.90 | 21.40 | 19.00 | 2.90 |
| | 76 | 22.30*** | ----- | ----- | ----- |
| | 82 | 21.80 | 21.25 | 18.40 | 3.40 |
| Average ± SD | | 23.33±1.36 | 22.66±1.12 | 20.49±1.47 | 2.98±0.75 |

*** – the weight of the mouse before the necropsy

**Table 14.** Tumour size in the mice from the EPI/OVCAR3 group during intravenous administration of EPI at a dose of 15 mg/kg bw (detailed results).

| Group | Mouse # | Tumour volume (mm³) | | | | Change in tumour volume (mm³) | Percentage change in tumour volume (%) | Volume of the tumour following dissection (mm³) |
|---|---|---|---|---|---|---|---|---|
| | | Before 1st administration | Before 2nd administration | One day after 2nd administration | Two days after 2nd administration | | | |
| EP-NPs in dose 15 mg/kg bw (group D) | 5 | 100.29 | 90.06 | 87.07 | 86.25 | -14.04 | -14.00 | 73.37 |
| | 15 | 164.36 | 160.79 | 155.78 | 135.44 | -28.92 | -17.60 | 85.22 |
| | 24 | 138.93 | 146.21 | 143.57 | 145.58 | 6.65 | +4.79 | 98.45 |
| | 29 | 116.49 | 106.88 | 103.86 | 119.24 | 2.75 | +2.36 | 67.46 |
| | 31 | 121.68 | 123.19 | 120.51 | 103.15 | -18.53 | -15.23 | 58.06 |
| | 32 | 100.05 | 114.78 | 110.86 | 105.54 | 5.49 | +5.49 | 65.33 |
| | 82 | 139.54 | 128.92 | 124.62 | 98.95 | -40.59 | -29.09 | 48.97 |
| | 76ᵉ | 107.13 | ---- | ---- | ---- | ---- | | 47.01 |
| Average ± SD | | 125.91 ± 23.33 | 124.40 ± 23.81 | 120.90 ±23.38 | 113.45 ± 21.09 | | | 67.98 ± 17.57 |

* - mouse died directly after EPI administration

**Table 15.** Results of macroscopic observations during necropsy of a mouse from the EPI/OVCAR3 group which received EPI at a dose of 15 mg/kg bw (mouse #5).

| External appearance of the mouse | Anal area dirty with faeces, mouse is cachectic, skin 0.21mm thick |
|---|---|
| **Organs** | **Macroscopic evaluation of internal organs** |
| Liver | Liver surface colour dark cherry to brown, consistency softer than normal. blood vessels highly filled with blood |
| Left kidney | Consistency softer than normal |
| Right kidney | Consistency softer than normal |
| Adrenal glands | No visible changes |
| Spleen | 1.6 cm x 0.4 cm x 0.1 cm in size. slightly smaller. dark brown in colour |
| Pancreas | No visible changes |
| Lung | No visible changes |
| Heart | No visible changes |
| Thymus | No visible changes |
| Intestines | Intestines filled with green food content, the content prevents an accurate assessment of the mucous membrane |
| Stomach | No visible changes |

(continued)

| External appearance of the mouse | Anal area dirty with faeces, mouse is cachectic, skin 0.21mm thick |
|---|---|
| **Organs** | **Macroscopic evaluation of internal organs** |
| Reproductive organs | No visible changes |
| Bladder | No visible changes |
| Brain | No visible changes |
| Tumour | 5.80 mm x 5.03 mm in size in the subcutaneous tissue, immobile about the skin. |

EPI administration to the mice from the EPI/OVCAR3 group resulted in the death of the mice a soon as on the 4th day after the first administration of the drug (after the second administration), one mouse died directly following the first administration of EPI, while the mice from the NPs/EPI/OVCAR3 group which received NPs-EPI in both concentrations survived to the end of the experiment.

Mice from the EPI/OVCAR3 group which received EPI exhibited severe inflammation and changes in the structure of the liver, as well as changes in the colour and structure of the spleen compared to the K1/OVCAR control group. Such changes were not observed in the NPsEPI/OVCAR3 group, which received NPs-EPI. Pure drug results in significant damage to local blood vessels, liver damage and the need to sacrifice the animals due to cachexia, which is not observed for epirubicin administered in the form encapsulated with a polysaccharide.

A significant decrease in the size of the induced ovarian tumour is observed in the mice from the NPsEPI/OVCAR3 group which received NPs-EPI in both concentrations (**Fig. 4 A** and **B**) compared to the size of tumours in the control group, with an increased decrease in tumour size observed when administering a higher dose of NPs-EPI.

Surprisingly, it was found that the use of an anthracycline encapsulated with a polysaccharide instead of its non-encapsulated form, e.g. NPs-EPI instead of EPI, reduces the drug toxicity sufficiently (despite not being a form of drug with an attached target cell targeting factor) to allow for intravenous administration of a higher dose of the drug and achieving a faster reduction of tumour volume.

### Example 4.

**Comparison of anti-tumour efficacy, safety, side effects of the therapy using encapsulated epirubicin NPs-EPI and EPI in free form in a mouse ovarian tumour model with implanted OVCAR3 line cells**

By **Example 1,** dextran nanoparticles NPs were prepared without epirubicin, nanoparticles with epirubicin encapsulated into dextran nanoparticles (NPs-EPI). The administration protocol was changed compared to **Example 3** to administer the dose of EPI or NPs-EPI every 3 days in an amount based on epirubicin 10 mg/kg bw or administer NPs as a control. Following **Example 3,** mice were obtained constituting an ovarian tumour test model, which were divided into 3 groups.

L **-** control group injected with NPs;
J -the test group which received epirubicin encapsulated in dextran nanoparticles (NPs-EPI) in the amount of 10 mg/kg bw
K - the test group which received epirubicin in the form of epirubicin hydrochloride in the amount of 10 mg/kg bw

The results obtained in the form of the size of tumour changes in individual mice and the average tumour volume are presented in **Tables 16** and **17** below.

**Table 16.** Changes in tumour volume after subsequent administrations of NPs-EPI, EPI and NPs to mice.

| Group | Mouse # | Volume of the tumour before 1st administration | Before 2nd administration | Before 3rd administration | Before 4th administration | Before 5th administration | Before 6th administration | Before 7th administration | Before necropsy | Volume of the tumour before the necropsy (mm³) |
|---|---|---|---|---|---|---|---|---|---|---|
| NPs 380 mg/kg bw (group L) | 132 | 112.33 | ↑4.44%[E] | ↑18.20% | ↑22.01% | ↑26.25% | ↑27.99% | ↑31.14% | ↑115.96%[G] | 242.59[G] |
| | 146 | 104.97 | ↑3.89% | ↑3.44% | ↑6.24% | ↑9.40% | ↑11.14% | ↑16.48% | ↑73.44% | 182.07 |
| | 147 | 100.36 | ↑30.70% | ↑34.45% | ↑35.12% | ↑36.22% | ↑21.82% | ↑29.92% | ↑50.35% | 150.89 |
| | 153 | 105.89 | ↓1.51%[E] | ↑65.82% | ↑82.17% | ↑102.04% | ↑174.10% | ↑336.91% | ↑498.42% | 633.67 |
| | 156 | 104.14 | ↓0.87% | ↓1.83% | ↓0.41% | ↑2.44% | ↑2.86% | ↑5.01% | ↑56.78% | 163.27 |
| | 163 | 118.69 | ↑13.31% | ↑24.80% | ↑39.27% | ↑59.73% | ↑66.85% | ↑72.42% | ↑209.38% | 367.20 |
| NPs-EPI 10 mg/kg bw (group J) | 136 | 254.35 | 0.00% | ↑7.38% | ↑8.17% | --- | --- | --- | ↑5.33%[H] | 267.91[H] |
| | 140 | 187.30 | ↓2.22% | ↓7.17% | ---[F] | --- | --- | --- | ↓11.71% | 165.37 |
| | 143 | 176.60 | ↓1.50% | ↓18.71% | ↓17.15% | --- | --- | --- | ↓5.83% | 166.30 |
| | 152 | 162.03 | ↑7.39% | ↑16.56% | ↑8.46% | --- | --- | --- | ↓3.04% | 157.11 |
| | 157 | 163.08 | ↑3.93% | ↓7.67% | ↓3.94% | --- | --- | --- | ↑19.18% | 194.36 |
| | 158 | 158.37 | ↓5,64% | ↓12.64% | ↓21.46% | ↓29.75% | ↓33.66% | ↓27.12% | ↓21.56% | 124.23 |
| | 159 | 172.90 | ↓12.34% | ↓13.02% | ↓21.31% | ↓41.97% | ↓42.37% | ↓36.59% | ↓33.65% | 114.72 |
| | 160 | 173.02 | ↓6.38% | ↓18.84% | ↓34.53% | --- | --- | --- | ↓43.54% | 97.69 |
| | 164 | 179.25 | ↓22.52% | ↓22.64% | ↓26.20% | ↓21.76% | --- | --- | ↓30.80% | 124.05 |
| NPs 10 mg/kg bw (group K) | 131 | 134.37 | ↑0.26% | ↓9.02% | --- | --- | --- | --- | ↓34.62%[I] | 87.85[I] |
| | 142 | 157.38 | ↓14.33% | ↓9.58% | --- | --- | --- | --- | ↓20.82% | 124.62 |
| | 148 | 137.41 | ↑7.21% | ↓28.86% | --- | --- | --- | --- | ↓33.27% | 91.69 |
| | 149 | 136.13 | ↓8.76% | ↓20.03% | --- | --- | --- | --- | ↓24.10% | 103.33 |
| | 162 | 150.89 | ↑53.65% | ↑40.73% | --- | --- | --- | --- | ↑57.69% | 237.94 |
| | 165 | 145.19 | ↓17.56% | ↓29.43% | --- | --- | --- | --- | ↓30.24% | 101.28 |

[E] percentage increase (↑) or decrease (↓) in tumour volume compared to the volume before the commencement of administration
[F] the mouse was sacrificed earlier for humanitarian reasons
[G] following 12 administrations in all animals in the group
[H] following 3 to 7 administrations in all animals in the group
[I] after 3 administrations in all animals in the group

**Table 17.** Comparison of changes in average tumour volume after subsequent administrations of NPs-EPI, EPI and NPs to mice.

| Group | Before 1st administration | Before 2nd administration | Before 3rd administration | Before 4th administration | Before 5th administration | Before 6th administration | Before 7th administration | Before necropsy |
|---|---|---|---|---|---|---|---|---|
| NPs (group L) | 107.73 ± 6.63 | 116.59 ± 13.57 ↑8.22%[J] | 133.71 ± 26.78 ↑24.12% | 141.02 ± 33.43 ↑30.90% | 150.60 ± 42.45 ↑39.79% | 163.01 ± 70.31 ↑51.31% | 196.10 ± 134.75 ↑82.03% | 289.95 ± 186.21 ↑169.15%[G] |
| NPs-EPI 10 mg/kg bw (group J) | 180.77 ± 29.08 | 172.98 ± 33.61 ↓4.31%[J] | 166.42 ± 43.60 ↓7.94% | 157.47 ± 51.33 ↓12.89% | 117.28 ↓35.12% | 102.35 ↓43.38% | 112.53 ↓37.75% | 156.86 ± 51.62 ↓13.23%[H] |
| EPI 10 mg/kg bw (group K) | 143.56 ± 9.22 | 148.77 ± 41.82 ↑3.63% | 131.00 ± 42.96 ↓8.75% | ---[K] | --- | --- | --- | 124.45 ± 57.05 ↓13.31%[I] |

[G] following 12 administrations in all animals in the group
[H] following 3 to 7 administrations in all animals in the group
[I] after 3 administrations in all animals in the group
[J] percentage increase (↑) or decrease (↓) in average tumour volume compared in the group to the volume before the commencement of administration
[K] all mice from the group were sacrificed earlier for humanitarian reasons

The results presented indicate that epirubicin encapsulated in dextran nanoparticles (NPs-EPI) has greater anti-tumour efficacy and less pronounced adverse effects than EPI. The higher toxicity of EPI is also demonstrated by the fact that as soon as following 3 administrations of drug doses, body weight decreased by 22.55% on average, while during the entire NPs-EPI treatment (3-7 doses), the body weight of the animals decreased by an average of 11.37%.

Thus, administration of NPs-EPI improves and prolongs the total survival time.

The analysis of average change in tumour volume showed that in animals treated with NPs-EPI, the tumours decreased on average by 4.31% as soon as after the administration of the first dose, while in animals receiving EPI during the same period the tumour was observed to increase by 3.63% on average. During the administration of NPs-EPI, increased efficacy was observed over time, which may confirm the gradual release of the drug from dextran nanoparticles. Microscopic images of internal organs indicated lower toxicity of NPs-EPI at a dose of 10 mg/kg bw versus the equivalent dose of EPI **(Table 18)**.

Table 18.

| Appearance of the organ | NPs-EPI at a dose of 10 mg/kg bw | EPI at a dose of 10 mg/kg bw |
|---|---|---|
| Liver | Hyperaemia, features of hepatocyte feathery degeneration | Hyperaemia, features of hepatocyte feathery degeneration, focal necrosis of hepatocytes |
| Reproductive organs | Moderate changes, lymphocytes in the myometrium | Severe lesions, endometrial hypertrophy, cystic lesions, the growth of Leydig cells within the ovary |
| Tumour | anaplastic lesions, pronounced tumour necrosis | anaplastic lesions, moderate tumour necrosis |

NPs-EPI administration observably resulted in increased survival of the mice, which allowed for the administration of the drug over a longer time, indicating that the encapsulation of EPI into polysaccharide nanoparticles improves the treatment efficacy with decreased toxicity.

The use of drugs from the group of anthracyclines, preferably epirubicin, daunorubicin, doxorubicin, idarubicin in the form of nanoparticles encapsulated with a polysaccharide, in particular, nanoparticles of epirubicin, daunorubicin, doxorubicin, idarubicin encapsulated with dextran reduces the toxicity of these compounds. The encapsulation of these anthracyclines with dextran allows for administering a higher intravenous dose of the drug, such as epirubicin, as demonstrated in tissue cultures of various tumours, the treatment of which uses these anthracyclines, as well as in a mouse model of ovarian tumour.

Due to similar curves of effect on tissue cultures of breast tumour, cervical cancer, ovarian cancer, pancreatic tumour, kidney tumour, lung tumour, colorectal tumour cancer lines obtained for epirubicin, daunorubicin, doxorubicin, idarubicin encapsulated both with dextran and other polysaccharides, which in the new form reduce toxicity of said drugs to the body (with the mechanism thereof not being clear, as these are particles without targeting particles attached), similar results of increased drug efficacy combined with reduced systemic toxicity with the possibility of administering a higher dose of the drug are obtained for epirubicin, daunorubicin, doxorubicin, idarubicin encapsulated with both dextran and other polysaccharides: cellulose and its derivatives, amylose, starch and heparin.

Surprisingly, it was found that the new form of anthracycline administration in the form of epirubicin, daunorubicin, doxorubicin, idarubicin encapsulated with a polysaccharide, particularly epirubicin encapsulated with dextran, allows for achieving a therapeutic effect while using lower doses of the drug, and it also allows for administering higher doses of the drug, longer therapy due to their reduced toxicity to the body, which provides improved efficacy of tumour treatment. Pure drug results in significant damage to local blood vessels, liver damage, as observed in an animal model, since in an exemplary EPI study it was necessary to sacrifice the animals due to cachexia much earlier than when administering NPs-EPI.

What is very promising is not only the unexpected achievement of the effect of reduced drug toxicity, but also the possibility of using encapsulated epirubicin, particularly encapsulated with dextran, to treat pancreatic tumour (observed significantly increased toxicity for PNAC1 cells - Fig 3 G.), in which treatment, according to the art, this chemotherapeutic agent was completely ineffective despite the attempts made.

**LITERATURE**

[200] J.V McGowan. R. Chung. A. Maulik. I Piotrowska. J. MalcolmWalker. D. Yellon1 Anthracycline Chemotherapy and Cardiotoxicity Cardiovascular Drugs Therapy (2017) 31:63-75

[201] G. Minotti. P. Menna. E. Salvatorelli. G. Cairo. L. Gianni. Anthracyclines: molecular advances and pharmacologic developments in antitumor activity and cardiotoxicity. Pharmacological reviews. 2004. 56(2). 185-229.

[202] D. Gewirtz. A critical evaluation of the mechanisms of action proposed for the antitumor effects of the anthracycline antibiotics adriamycin and daunorubicin. Biochemical pharmacology. 1999. 57(7). 727-741

[203] T. Mosmann. Rapid colorimetric assay for cellular growth and survival: application to proliferation and cytotoxicity assays. Journal of immunological methods. 1983. 65(1-2). 55-63.

[204] ISO standards 10993-5: 2009 Biological evaluation of medical devices- Part 5: Tests for *In vitro* cytotoxicity

[205]    http://www.thermofisher.com/pl/en/home/references/protocols/cell-culture/mtt-assay-protocol/vybrant-mtt-cell-proliferation-assay-kit.htm

[206] R. I. Freshney. Cytotoxicity. Culture of animal cells. A Manual of Basic Technique. Wiley-Blackwell. 2005. ISBN 9780470528129

[207] M. Volkova. R. Russell Anthracycline Cardiotoxicity: Prevalence. Pathogenesis and Treatment. Current Cardiology Reviews. 2011. 7. 214-220

[208] D. Bobo. K. Robinson. J. Islam. K. Thurecht. S. Corrie. Nanoparticle-Based Medicines: A Review of FDA-Approved Materials and Clinical Trials to Date Pharmaceutical Research. 2016 Oct;33(10):2373-87.

## Claims

1. An anthracycline encapsulated with a polysaccharide for use in the treatment of tumour, **characterised in that** the anthracycline is epirubicin the tumour is ovarian cancer and polysaccharide is dextran.

2. The anthracycline encapsulated with the polysaccharide for use in the treatment of tumour according to claim 1, **characterised in that in that** the particles of the anthracycline encapsulated with the polysaccharide have in the hydrated state an average size in the range of 10-500 nm, more preferably 50-200 nm, most preferably 70-160 nm.

## Patentansprüche

1. Ein mit einem Polysaccharid eingekapseltes Anthracyclin zur Verwendung bei der Behandlung eines Tumors, **dadurch gekennzeichnet, dass** das Anthracyclin Epirubicin ist, der Tumor Eierstockkrebs ist und das Polysaccharid Dextran ist.

2. Das mit dem Polysaccharid eingekapselte Anthracyclin zur Verwendung bei der Behandlung von Tumoren nach Anspruch 1, **dadurch gekennzeichnet, dass** die mit dem Polysaccharid eingekapselten Anthracyclinpartikel im hydratisierten Zustand eine durchschnittliche Größe im Bereich von 10-500 nm, vorzugsweise 50-200 nm, besonders bevorzugt 70-160 nm, aufweisen.

## Revendications

1. Une anthracycline encapsulée avec un polysaccharide destinée à être utilisée dans le traitement d'une tumeur, **caractérisée en ce que** l'anthracycline est l'épirubicine, la tumeur est un cancer de l'ovaire et le polysaccharide est le dextrane.

2. L'anthracycline encapsulée avec le polysaccharide destinée à être utilisée dans le traitement d'une tumeur selon la revendication 1, **caractérisée en ce que** les particules de l'anthracycline encapsulée avec le polysaccharide ont, à l'état hydraté, une taille moyenne comprise entre 10 et 500 nm, préférablement entre 50 et 200 nm, et le plus préférablement entre 70 et 160 nm.

**FIG. 1**

**Fig. 2**

Fig. 2 – continued

**Fig. 3**

EP 3 761 955 B1

**Fig. 4**

| Changes in the size of tumours | Changes in body weight |
|---|---|

30

## REFERENCES CITED IN THE DESCRIPTION

**Patent documents cited in the description**

- PL 221351
- KR 20170093400 A

- PL 221251


**Non-patent literature cited in the description**

- **IGA WASIAK et al.** Dextran Nanoparticle Synthesis and Properties. *PLOS ONE*, vol. 11
- **AGARWAL A et al.** Dextran conjugated dendritic nanoconstructs as potential vectors for anti-cancer agent. *BIOMATERIALS*, vol. 30, 3588-3596
- **J.V MCGOWAN** ; **R. CHUNG** ; **A. MAULIK** ; **I PIOTROWSKA** ; **J. MALCOLMWALKER** ; **D. YELLON1**. Anthracycline Chemotherapy and Cardiotoxicity. *Cardiovascular Drugs Therapy*, 2017, vol. 31, 63-75
- **G. MINOTTI** ; **P. MENNA** ; **E. SALVATORELLI** ; **G. CAIRO** ; **L. GIANNI**. Anthracyclines: molecular advances and pharmacologic developments in antitumor activity and cardiotoxicity. *Pharmacological reviews*, 2004, vol. 56 (2), 185-229
- **D. GEWIRTZ**. A critical evaluation of the mechanisms of action proposed for the antitumor effects of the anthracycline antibiotics adriamycin and daunorubicin. *Biochemical pharmacology*, 1999, vol. 57 (7), 727-741

- **T. MOSMANN**. Rapid colorimetric assay for cellular growth and survival: application to proliferation and cytotoxicity assays. *Journal of immunological methods*, 1983, vol. 65 (1-2), 55-63
- Cytotoxicity. **R. I. FRESHNEY**. Culture of animal cells. A Manual of Basic Technique. Wiley-Blackwell, 2005
- **M. VOLKOVA** ; **R. RUSSELL**. Anthracycline Cardiotoxicity: Prevalence. Pathogenesis and Treatment. *Current Cardiology Reviews*, 2011, vol. 7, 214-220
- **D. BOBO** ; **K. ROBINSON** ; **J. ISLAM** ; **K. THURECHT** ; **S. CORRIE**. Nanoparticle-Based Medicines: A Review of FDA-Approved Materials and Clinical Trials to Date. *Pharmaceutical Research*, October 2016, vol. 33 (10), 2373-87